(19) Europäisches Patentamt
European Patent Office
Office européen des brevets

(11) **EP 2 567 238 B1**

(12) **FASCICULE DE BREVET EUROPEEN**

(45) Date de publication et mention
de la délivrance du brevet:
**24.06.2015 Bulletin 2015/26**

(21) Numéro de dépôt: **11723563.0**

(22) Date de dépôt: **04.05.2011**

(51) Int Cl.:
***G01N 33/68*** (2006.01)    ***G01N 33/564*** (2006.01)

(86) Numéro de dépôt international:
**PCT/FR2011/051012**

(87) Numéro de publication internationale:
**WO 2011/138561 (10.11.2011 Gazette 2011/45)**

(54) **PROCÉDÉ DE MESURE DE L'ACTIVATION DU COMPLÉMENT PAR DES IgG**

VERFAHREN ZUM MESSEN VON IgG-VERMITTELTER KOMPLEMENTAKTIVIERUNG

METHOD FOR MEASURING IgG-MEDIATED COMPLEMENT ACTIVATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.05.2010 FR 1053515**

(43) Date de publication de la demande:
**13.03.2013 Bulletin 2013/11**

(73) Titulaire: **Laboratoire Français du Fractionnement et des Biotechnologies 91940 Les Ulis (FR)**

(72) Inventeurs:
• **DHAINAUT, Frédéric 91870 Boissy Le Sec (FR)**
• **PERRET, Gérald 94600 Choisy Le Roi (FR)**

(74) Mandataire: **Cabinet Plasseraud 52, rue de la Victoire 75440 Paris Cedex 09 (FR)**

(56) Documents cités:
**US-A- 5 612 033    US-A- 5 972 632**

• **SPYCHER M ET AL: "In vitro comparison of the complement-scavenging capacity of different intravenous immunoglobulin preparations", VOX SANGUINIS, vol. 97, no. 4, 2009, pages 348-354, XP007914912, ISSN: 0042-9007**
• **LEON G ET AL: "Anticomplementary activity of equine whole IgG antivenoms: comparison of three fractionation protocols", TOXICON, ELMSFORD, NY, US LNKD- DOI: 10.1016/J.TOXICON.2004.07.025, vol. 45, no. 1, 1 janvier 2005 (2005-01-01), pages 123-128, XP004667169, ISSN: 0041-0101**
• **BUCHACHER A ET AL: "Anticomplementary activity of IVIG concentrates - important assay parameters and impact of IgG polymers", VOX SANGUINIS, vol. 98, no. 3, avril 2010 (2010-04), pages E209-E218, XP007914915, ISSN: 0042-9007**
• **KAPP A ET AL: "DETECTION OF COMPLEMENT ACTIVATION IN HUMAN SERUM USING COMPLEMENT C-5A-INDUCED CHEMILUMINESCENCE OF HUMAN GRANULOCYTES", ARCHIVES OF DERMATOLOGICAL RESEARCH, vol. 278, no. 1, 1985, pages 41-43, XP9138711, ISSN: 0340-3696**
• **RAMASAMY INDRA ET AL: "Measurement of anticomplementary activity in therapeutic intravenous immunoglobulin preparations", BIOLOGICALS, vol. 25, no. 1, 1997, pages 87-92, XP007914920, ISSN: 1045-1056**

**EP 2 567 238 B1**

Printed by Jouve, 75001 PARIS (FR)

**Description**

L'invention concerne un procédé de mesure de l'activation du complément par des immunoglobulines G (IgG), notamment des immunoglobulines G intraveineuses humaines (IVIG). Le procédé permet de sélectionner les IVIG en fonction de leur effet sur le complément.

Les IVIG sont un concentré d'immunoglobulines G non agrégées préparées à partir d'un pool de plasma humain. Leur fonction est intacte, et leur pureté la plus élevée possible (> 99,9%).

Les IVIG modulent, par des mécanismes moléculaires sélectifs et souvent distincts, certains processus biologiques impliqués dans la réponse immune innée ou acquise. Ces derniers incluent :

- un blocage fonctionnel des récepteurs Fc du système réticulo-endothélial. Cela permet notamment de traiter les cytopénies auto-immunes périphériques, comme le purpura thrombocytopénique idiopathique ;
- la neutralisation d'auto-anticorps et l'inhibition de leur production. Les IVIG contiennent en effet des anticorps anti-idiotypiques. Une telle activité neutralisante ou inhibitrice a été démontrée par exemple pour des auto-anticorps contre le facteur VIII, l'ADN, le facteur intrinsèque, la thyréoglobuline et les ANCA (Anti-Neutrophil Cytoplasmic Antibodies) ;
- la modulation de la production de cytokines (dont l'IL-1, -2, -3, -4, -5, -10, le TNF-a, et le GM-CSF) et de leurs antagonistes (comme IL-1 RA), dont la résultante est une activité anti-inflammatoire ;
- l'activation du complément ;
- ou encore l'activation ou le blocage fonctionnel du récepteur de mort cellulaire Fas (CD95).

Aujourd'hui, l'administration d'IVIG est devenue un geste thérapeutique comportant très peu de risques ou d'effets secondaires (< 1%). Ces derniers sont mineurs grâce à l'amélioration constante de la qualité de ces produits.

La plupart des effets secondaires de nature mineure et transitoire surviennent dans la première heure de perfusion ; ils comprennent des myalgies, céphalées, bouffées de chaleur, sensations d'oppression, dorsalgies, nausées, vomissements, bronchospasmes, ou encore changement de pouls et/ou tension artérielle. Ils sont vraisemblablement la conséquence d'une certaine agrégation des immunoglobulines, de la formation de complexes antigène-anticorps et l'activation du complément, ou de sucres présents dans les IVIG dans le but de prévenir leur agrégation. Ces effets secondaires sont en général jugulés en diminuant la vitesse de perfusion ou en administrant rapidement des stéroïdes ou antihistaminiques voire des sympathicomimétiques.

Dans certains cas cependant, une insuffisance rénale aiguë et/ou une anémie hémolytique peuvent survenir. Dans des cas extrêmes, une réaction anaphylactique peut survenir, et ceci particulièrement chez des patients ayant une déficience en IgA sériques.

La diminution des effets secondaires des IVIG, si mineurs soient-ils, reste donc une préoccupation majeure dans le développement de ces molécules.

Différentes méthodes sont actuellement disponibles pour évaluer ces effets secondaires, par exemple le test d'AAC (activité anticomplémentaire) de la pharmacopée européenne, également appelé essai d'activité anticomplémentaire de l'immunoglobuline ; ou encore le test de Coombs. Le test d'AAC est complexe et fait intervenir des réactifs difficilement normalisables. En effet, la détermination de l'activité anticomplémentaire (AAC) de l'immunoglobuline est effectuée par incubation d'une quantité donnée de l'immunoglobuline à examiner avec une quantité donnée de complément de cobaye suivie du titrage du complément restant. Le complément restant est mesuré par la lyse d'érythrocytes de mouton sensibilisés par des anticorps de lapin.

Quant au test de Coombs, il permet de vérifier l'absence d'immunoglobuline pouvant se fixer sur les hématies et pouvant entraîner l'agglutination et la lyse des hématies.

SPYCHER M ET AL: "In vitro comparison of the complement-scavenging capacity of different intravenous immunoglobulin preparations", VOX SANGUINIS, vol. 97, no. 4, 2009, p.348-354, décrit la comparaison des propriétés de piégeage du complément par différentes IVIG. Cette comparaison est effectuée selon plusieurs protocoles, visant le piégeage de C3b/iC3b par les IVIG, la liaison du C3b activé aux IVIG, et la génération de C5a par les IVIG.

Il existe donc un besoin de disposer de tests suffisamment sensibles pour sélectionner différentes qualités d'IVIG, qui soient faciles à mettre en place pour une utilisation de routine. Il existe également un besoin de disposer de tests pertinents et prédictifs pour l'homme. Ces tests doivent en particulier démontrer une bonne qualité et une sécurité suffisante des IVIG pour le patient.

C'est pourquoi la Demanderesse s'est attachée à la mise au point d'un nouveau procédé *in vitro* permettant d'évaluer l'action des IgG, notamment des IVIG, sur l'activation du complément. Ce procédé permet de sélectionner facilement les IgG ayant le moins d'effets secondaires possibles, et ce avec une sensibilité suffisante pour discriminer différentes qualités d'IgG. Ce procédé utilise du sérum humain, et est par conséquent prédictif du résultat sur l'homme.

La présente invention a donc pour objet un procédé de mesure de l'activation du complément par des immunoglobulines G, comprenant les étapes suivantes :

a) préparation d'un échantillon A d'immunoglobulines G (IgG), et d'un échantillon B comprenant du sérum natif, ledit sérum natif étant éventuellement dilué dans un tampon de dilution ;

b) mélange de l'échantillon A avec l'échantillon B dans un ratio (quantité d'IgG dans A, en gramme) : (volume de sérum natif dans B, en litre) compris entre 30 et 75, à une température comprise entre 2°C et 6°C, puis incubation du mélange réactionnel obtenu à une température comprise entre 35°C et 40°C pendant un temps compris entre 30 minutes et 2 heures ;

c) refroidissement du mélange réactionnel obtenu à la fin de l'étape b) à une température comprise entre 0°C et 4°C et en présence d'EDTA ;

d) mesure de la quantité de fragment C5a dans le mélange réactionnel refroidi obtenu en c).

Bien évidemment, le procédé selon l'invention est un procédé *in vitro*.

Ce procédé mesure l'activation du complément par des IgG, de préférence des IgG humaines, de préférence des IVIG. Aussi de préférence, l'échantillon A est un échantillon d'IVIG.

L'étape a) du procédé selon l'invention comprend la préparation d'un échantillon A d'IgG. Cet échantillon A peut notamment être obtenu à partir d'IVIG commerciales, comme Privigen de CSL Behring AG, Gamunex de Bayer Corporation, Intratect de Biottest Pharma GmbH, Kiovig de Baxter AG ou Octagam d'Octapharma. La préparation peut être sous forme liquide ou lyophilisée, comme Sandoglobulin de CSL Behring AG.

L'échantillon A peut également être obtenu à partir d'IVIG non commerciales, comme de nouvelles IVIG (IgNG).

Les IgG, de préférence IVIG, peuvent être diluées dans un milieu tampon classiquement utilisé, comme le tampon phosphate salin/sérum albumine bovine (i.e. PBS-BSA). De préférence, les IgG, notamment les IVIG, sont diluées dans un tampon comprenant de la BSA.

L'étape a) comprend également la préparation d'un échantillon B comprenant du sérum natif.

Par sérum, on entend le plasma sanguin débarrassé de la fibrine. Par « sérum natif », on entend un sérum non dilué. Le sérum natif est de préférence du sérum humain natif. Le sérum natif de l'échantillon B peut provenir de différents donneurs, ayant différents groupes sanguins. De préférence, le sérum natif est un sérum humain de groupe sanguin AB+.

De préférence, le sérum natif est conservé congelé jusqu'à utilisation, par exemple à une température de -80°C. Lors de l'utilisation, il est alors doucement décongelé jusqu'à la température de mélange.

L'échantillon B peut contenir uniquement du sérum natif ; alternativement, l'échantillon B comprend du sérum natif dilué dans un tampon de dilution.

Un tel tampon de dilution est classique, et peut être choisi parmi le tampon phosphate salin (PBS), le PBS-BSA.

De préférence, le tampon de dilution comprend de la BSA ; de préférence, le tampon de dilution est le PBS-BSA.

De préférence, l'échantillon A comprend des IgG diluées dans un tampon identique au tampon de dilution de l'échantillon B.

Une fois les échantillons A et B préparés, ces derniers sont mélangés puis incubés dans des conditions spécifiques lors de l'étape b) du procédé selon l'invention.

En effet, le mélange des échantillons A et B se fait :

- dans un ratio (quantité d'IgG dans A, en gramme) : (volume de sérum natif dans B, en litre) compris entre 30 et 75, et
- à une température comprise entre 2°C et 6°C.

Le ratio (quantité d'IgG dans A, en gramme) : (volume de sérum natif dans B, en litre) définit la quantité d'IgG qui agit sur un volume de sérum natif. La quantité d'IgG présente dans A est déterminée en gramme, et le volume de sérum natif présent dans B est déterminé en litre.

Par exemple, si l'échantillon A correspond à 20 $\mu$l d'une solution d'IgG à 100g/l, alors la quantité d'IgG présente dans A est de $100*20*10^{-6} = 2.10^{-3}$g. De même, si l'échantillon B correspond à 80 $\mu$l d'une solution de sérum natif dilué à 25% dans un tampon de dilution, alors le volume de sérum natif dans B est de $80*10^{-6}*0.25 = 2*10^{-5}$l (soit 20 $\mu$l). Dans ce cas, le ratio (quantité d'IgG dans A, en gramme) : (volume de sérum natif dans B, en litre) est de 2/0.02 soit 100.

De préférence, le ratio (quantité d'IgG dans A, en gramme) : (volume de sérum natif dans B, en litre) est compris entre 35 et 70, de préférence entre 40 et 65, de préférence entre 45 et 60, de préférence entre 45 et 55, de préférence est d'environ 50.

La température de mélange est comprise entre 2°C et 6°C, de préférence entre 3°C et 5°C, de préférence est d'environ 4°C. Le mélange peut ainsi se faire dans un récipient, par exemple un tube à essai, conservé dans la glace jusqu'à incubation.

Si plusieurs échantillons A et B doivent être testés, tous les mélanges réactionnels sont réalisés à une température comprise entre 2°C et 6°C, de préférence à 4°C, puis l'incubation est réalisée simultanément de façon uniforme.

Une fois le mélange des échantillons A et B obtenu (ci-après mélange réactionnel), le mélange réactionnel est incubé à une température comprise entre 35°C et 40°C pendant un temps compris entre 30 minutes et 2 heures. De préférence, le mélange réactionnel est incubé à une température d'environ 37°C, pendant un temps compris entre 45 minutes et 1 h30, de préférence compris entre 45 minutes et 1 h, de préférence environ 1 h.

L'incubation se fait avec des appareils standards, par exemple à l'étuve ou dans un bain-marie thermostaté.

Le mélange réactionnel est ensuite refroidi lors d'une étape c), afin de stopper les réactions biologiques se déroulant pendant l'incubation. Ce refroidissement du mélange réactionnel obtenu à la fin de l'étape b) se fait à une température comprise entre 0°C et 4°C en présence d'EDTA. L'étape c) peut ainsi se dérouler en mettant les tubes à essai comprenant les mélanges réactionnels incubés dans la glace, et en ajoutant de l'EDTA en quantité suffisante. De préférence, l'EDTA est utilisé à une concentration comprise entre 50 mM et 200 mM, de préférence d'environ 100 mM.

Dans le mélange réactionnel obtenu en fin d'étape c), la quantité de fragment C5a du complément est alors mesurée (étape d)).

L'étape d) peut se faire par toute méthode de mesure classiquement utilisée, et notamment à l'aide de kits commerciaux. Par exemple, elle peut se faire par ELISA, à l'aide de kits commerciaux de détection de la protéine C5a, comme ceux vendus par Quidel ou R&D system ou Abcam.

La quantité de fragment C5a libérée au cours du procédé selon l'invention est déterminante pour évaluer les éventuels effets secondaires des IgG testées. Plus cette quantité est importante, plus les IgG auront d'effets secondaires indésirables.

Afin d'évaluer la qualité des IgG de la façon la plus précise et sensible possible, le procédé selon l'invention comprend avantageusement une étape supplémentaire e) de comparaison de la quantité obtenue en d) (i.e. quantité de fragment C5a libérée grâce à l'échantillon A et donc grâce aux IgG testées) avec la quantité obtenue en substituant l'échantillon A par un témoin positif.

Par témoin positif, on entend un échantillon responsable d'une activation maximale du complément, et donc d'une libération maximale de fragment C5a. Ce témoin positif sert ainsi de référence, car il correspond à 100% d'activation du complément.

Le témoin positif est avantageusement choisi parmi les échantillons de lipopolysaccharides (LPS), et notamment parmi les LPS d'Escherichia Coli (E.Coli) de sérotype 0127 :B8 purifiés par chromatographie échangeuse d'ions, les LPS d'E.Coli de sérotype K-235, les LPS de Salmonella enterica de sérotype typhimurium, les LPS de Klebsiella pneumoniae, les LPS d'E.Coli de sérotype 0127 :B8 extraits à l'acide trichloroacétique, les LPS de mutants de Shigella flexneri, et les LPS d'E.Coli de sérotype 0127 :B8 purifiés par extraction au phénol.

De préférence, le témoin positif est constitué par les LPS d'E.Coli de sérotype 0127 :B8 purifiés par extraction au phénol. Ces LPS activent en effet fortement la libération de fragment C5a, et ce de façon reproductible.

De préférence, le procédé selon l'invention comprend également une étape supplémentaire f) de comparaison de la quantité obtenue en d) (i.e. quantité de fragment C5a libérée grâce à l'échantillon A et donc grâce aux IgG testées) avec la quantité obtenue en substituant l'échantillon A par un contrôle négatif (ou témoin négatif). Par contrôle ou témoin négatif, on entend un échantillon responsable de la libération d'une quantité basale de fragment C5a. Ce contrôle négatif correspond notamment à un échantillon comprenant uniquement le tampon de dilution de l'échantillon B, par exemple le tampon PBS ou le tampon PBS-BSA.

Cette étape f) peut être réalisée en même temps, antérieurement ou postérieurement à l'étape e).

De préférence, le procédé selon l'invention comprend, comme étape e) ci-dessus, une étape de mesure du pourcentage d'activation du complément, obtenue par la formule suivante :

[test - contrôle négatif] / [activation maximale du complément - contrôle négatif] * 100

dans laquelle :

« test » est la quantité de fragment C5a mesurée dans le mélange réactionnel testé ;
« contrôle négatif » est la quantité de fragment C5a mesurée dans le mélange réactionnel dans lequel l'échantillon A est remplacé par le tampon de dilution ;
« activation maximale du complément » est la quantité de fragment C5a mesurée dans le mélange réactionnel dans lequel l'échantillon A est remplacé par des LPS d'E.Coli de sérotype 0127 :B8 purifiés par extraction au phénol.

De préférence, lorsqu'on utilise un témoin positif constitué par des LPS, notamment les LPS d'E.Coli de sérotype 0127 :B8 purifiés par extraction au phénol, le ratio (quantité de LPS, en gramme) : (volume de sérum natif dans B, en litre) est compris entre 6 et 15, de préférence entre 7 et 14, de préférence entre 8 et 13, de préférence le ratio est d'environ 10.

Les exemples suivants illustrent l'invention sans toutefois en limiter la portée.

### EXEMPLE 1 : procédé général de mesure de l'activation du complément par des IgG

Matériel :

Sérum humain natif : obtenu en mélangeant le sérum natif de 4 donneurs de type AB, et conservé à -80°C. Le sérum est délicatement décongelé à 4°C avant utilisation. Pour le test ELISA, les anticorps monoclonaux anti-C5a humain (pour le coating) et les anticorps polyclonaux anti-C5a humain biotinylés (pour la révélation) proviennent de R&D system.

Test de libération de C5a :

Etape a) : préparation de 20$\mu$l d'IgG testées à 50mg/ml dans un tampon PBS-BSA 1% (échantillon A) et de 80$\mu$l de sérum humain natif dilué à 25% dans du PBS-BSA 1% (échantillon B).

Etape b) : mélange de l'échantillon A avec l'échantillon B. Tous les tubes de mélange sont maintenus dans un mélange eau/glace jusqu'à incubation.

Pour chaque échantillon, 5 tests indépendants d'activation de C5a sont réalisés :

- L'activation maximale du complément (témoin positif) est obtenue en remplaçant l'échantillon A comprenant les IgG testées par des LPS E.Coli de sérotype 0127 :B8 purifiés par extraction au phénol (10mg/ml) ;
- Un témoin négatif est réalisé avec uniquement du tampon PBS-BSA.

Les mélanges sont incubés 1h à 37°C.

Etape c) : les réactions sont stoppées en plaçant les tubes dans la glace et en ajoutant 1 $\mu$l d'EDTA à 100mM.

Etape d) : après incubation et refroidissement, la libération du fragment C5a est mesurée en triplicate pour chaque tube par un test ELISA :

- Dilution de chaque mélange : 1/50 dans du PBS/BSA 1%
- Anticorps pour le coating : 0,4$\mu$g/ml - 100$\mu$l / Anticorps pour la révélation: 100ng/ml - 100$\mu$l

La densité optique est mesurée pour chaque mélange grâce à un lecteur de microplaques (Perkin) adapté au système de révélation associé à l'anticorps. Il peut s'agir d'un lecteur dans le visible dans le cadre d'une révélation classique HRP-OPD comme d'un lecteur de fluorescence si l'anticorps est greffé avec un groupement fluorescent.
Résultats: le pourcentage d'activation du complément est obtenu par la formule suivante :

**[test - contrôle négatif] / [activation maximale du complément - contrôle négatif] * 100**

Pour chaque mélange, la moyenne obtenue pour les 5 tests indépendants et l'écart-type sont mesurés.

### EXEMPLE 2 : évaluation de l'activation maximale du complément par différents LPS

Le protocole de l'exemple 1 est utilisé pour mesurer l'activation maximale du complément des différents LPS suivants :

LPS A: LPS d'E.Coli de sérotype 0127:B8 purifiés par chromatographie échangeuse d'ions
LPS B: LPS d'E.Coli de sérotype K-235
LPS C: LPS de Salmonella enterica de sérotype typhimurium
LPS D: LPS de Klebsiella pneumoniae
LPS E: LPS d'E.Coli de sérotype 0127:B8 extraits à l'acide trichloroacétique
LPS F: LPS de mutant de Shigella flexneri
LPS référence: LPS d'E.Coli de sérotype 0127:B8 purifiés par extraction au phénol

La quantité de fragment C5a libéré est évaluée aux jours 1 à 5 pour chaque mélange.

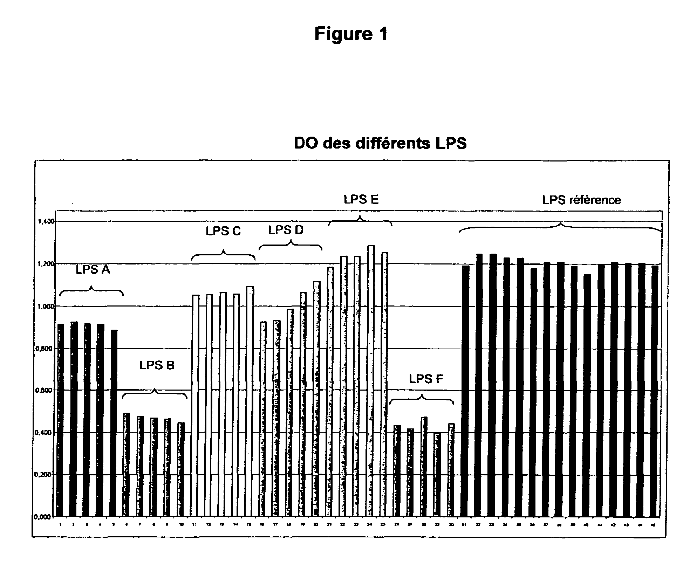

Les résultats sont présentés en Figure 1. Pour chacun des LPS A à F figurent successivement les densités optiques (DO) obtenues à J1, J2, J3, J4 et J5.

Pour le LPS de référence, les DO obtenues lors de chaque mesure pour les 5 jours sont données successivement (i.e. J1 : 1ère mesure, 2ème mesure et 3ème mesure du triplicate ; J2 : 1ère mesure, 2ème mesure et 3ème mesure du triplicate... jusqu'à J5 : 1ère mesure, 2ème mesure et 3ème mesure du triplicate).

Les résultats démontrent que le LPS de référence fait partie de ceux qui activent fortement C5a, et ce de façon constante au cours du temps.

### EXEMPLE 3 : mesure de l'activation du complément par des IVIG

Protocole :

Le protocole de l'exemple 1 est utilisé pour mesurer le pourcentage d'activation d'une IgNG et de différentes IVIG commerciales.

L'IgNG (ClairYg) est obtenue selon le procédé développé par la Demanderesse dans l'exemple 1 de la demande de brevet internationale WO 2007/077365.

4.IVIG liquides commerciales sont également testées, qui sont :

IVIG1 = Octagam® commercialisées par Octapharma ;
IVIG2 = Gammagard® commercialisées par Baxter ;
IVIG3 = Gamunex® commercialisées par Talecris ;
IVIG4 = Privigen® commercialisées par CSL Behring.

Pour le test ELISA, des plaques micropuits sont recouvertes avec $100\mu$l d'anticorps anti-C5a à $0.4\mu$g/ml dans un tampon comprenant du carbonate 0.1 M, pH 9.6, toute une nuit à 4°C. Après saturation avec PBS + 1% BSA, les échantillons et témoins/contrôles sont dilués dans le tampon de saturation, déposés sur plaque et incubés 2h à 20°C. L'anticorps polyclonal biotinylé est ensuite ajouté et révélé avec de la streptavidine couplée à une péroxydase. L'absorbance à 492 nm est mesurée avec un lecteur ELISA (Bio-Tek instrument). Le Pourcentage d'Activation du Complément est obtenu selon la formule suivante:

$$[test - contrôle négatif] / [activation maximale du complément - contrôle négatif] * 100$$

Pour chaque échantillon le résultat correspond à la moyenne des 5 activations indépendantes.

Résultats :

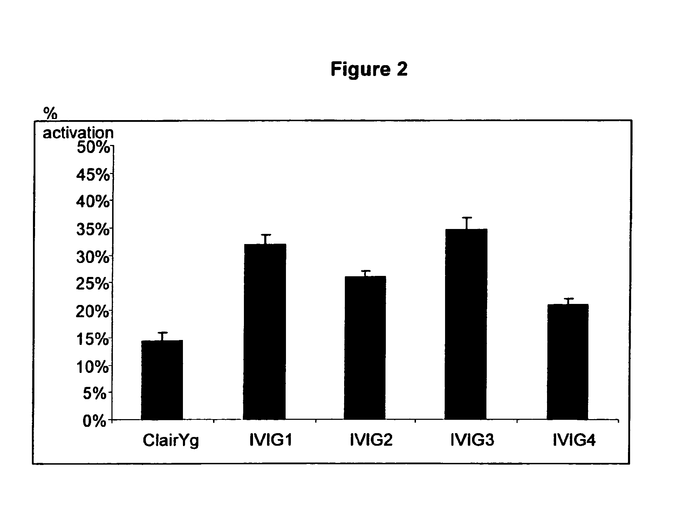

Les résultats sont présentés en Figure 2.

Il est clair que le pourcentage d'activation de C5a par ClairYg est significativement le plus faible (environ 15%), ce qui suggère, de manière significative, une meilleure tolérance de cette IVIG comparé aux 4 IVIG commerciales.

## Revendications

1. Procédé de mesure de l'activation du complément par des immunoglobulines G, comprenant les étapes suivantes :

   a) préparation d'un échantillon A d'immunoglobulines G (IgG), et d'un échantillon B comprenant du sérum natif, ledit sérum natif étant éventuellement dilué dans un tampon de dilution ;
   b) mélange de l'échantillon A avec l'échantillon B dans un ratio "quantité d'IgG dans A, en gramme" : "volume de sérum natif dans B, en litre" compris entre 30 et 75, à une température comprise entre 2°C et 6°C, puis incubation du mélange réactionnel obtenu à une température comprise entre 35°C et 40°C pendant un temps compris entre 30 minutes et 2 heures ;
   c) refroidissement du mélange réactionnel obtenu à la fin de l'étape b) à une température comprise entre 0°C et 4°C et en présence d'EDTA ;
   d) mesure de la quantité de fragment C5a dans le mélange réactionnel refroidi obtenu en c).

2. Procédé selon la revendication 1, **caractérisé en ce que** le ratio "quantité d'IgG dans A, en gramme" : "volume de sérum natif dans B, en litre" de l'étape b) est compris entre 35 et 70.

3. Procédé selon la revendication 2, **caractérisé en ce que** le ratio "quantité d'IgG dans A, en gramme" : "volume de sérum natif dans B, en litre" de l'étape b) est compris entre 40 et 65.

4. Procédé selon la revendication 2 ou 3, **caractérisé en ce que** le ratio "quantité d'IgG dans A, en gramme" : "volume de sérum natif dans B, en litre" de l'étape b) est compris entre 45 et 60.

5. Procédé selon l'une des revendications 1 à 4, **caractérisé en ce que** l'incubation de l'étape b) se fait à une température d'environ 37°C, pendant un temps compris entre 45 minutes et 1 h.

6. Procédé selon l'une quelconque des revendications 1 à 5, **caractérisé en ce que** l'EDTA est utilisé à l'étape c) à une concentration comprise entre 50 mM et 200 mM.

7. Procédé selon la revendication 6, **caractérisé en ce que** l'EDTA est utilisé à l'étape c) à une concentration d'environ 100 mM.

8. Procédé selon l'une quelconque des revendications 1 à 7, **caractérisé en ce que** l'échantillon A est un échantillon d'IVIG.

9. Procédé selon l'une quelconque des revendications 1 à 8, **caractérisé en ce que** le sérum natif de l'échantillon B est un sérum humain de groupe sanguin AB+.

10. Procédé selon l'une quelconque des revendications 1 à 9, **caractérisé en ce qu'**il comprend une étape e) de comparaison de la quantité obtenue en d) avec la quantité obtenue en substituant l'échantillon A par des lipopolysaccharides (LPS) d'E.Coli de sérotype 0127 :B8 purifiés par extraction au phénol.

11. Procédé selon l'une quelconque des revendications 1 à 9, **caractérisé en ce qu'**il comprend une étape e) de mesure du pourcentage d'activation du complément, obtenue par la formule suivante :

[test - contrôle négatif] / [activation maximale du complément - contrôle négatif] * 100
dans laquelle :

« test » est la quantité de fragment C5a mesurée dans le mélange réactionnel testé ;
« contrôle négatif » est la quantité de fragment C5a mesurée dans le mélange réactionnel dans lequel l'échantillon A est remplacé par le tampon de dilution ;
« activation maximale du complément » est la quantité de fragment C5a mesurée dans le mélange réactionnel dans lequel l'échantillon A est remplacé par des lipopolysaccharides (LPS) d'E.Coli de sérotype 0127 :B8 purifiés par extraction au phénol.

12. Procédé selon la revendication 10 ou 11, **caractérisé en ce que** le ratio "quantité de LPS, en gamme" : "volume de sérum natif dans B, en litre" est compris entre 6 et 15.

13. Procédé selon la revendication 12, **caractérisé en ce que** le ratio "quantité de LPS, en gramme" : "volume de sérum natif dans B, en litre" est compris entre 7 et 14.

14. Procédé selon l'une des revendications 12 ou 13, **caractérisé en ce que** le ratio "quantité de LPS, en gramme" : "volume de sérum natif dans B, en litre" est compris entre 8 et 13.

15. Procédé selon l'une quelconque des revendications 1 à 14, **caractérisé en ce que** l'étape d) de mesure de la quantité de fragment C5a est réalisée par un test ELISA.

**Patentansprüche**

1. Verfahren zur Messung der Komplementaktivierung durch Immunglobuline G, umfassend die folgenden Schritte:

a) Herstellung einer Probe A von Immunglobulinen G (IgG), und einer Probe B, die natives Serum umfasst, wobei das native Serum gegebenenfalls verdünnt wird in einem Verdünnungspuffer;

b) Mischen der Probe A mit der Probe B in einem Verhältnis der Menge des IgG in A in Gramm : Volumen natives Serum in B in Liter, umfassend zwischen 30 und 75, bei einer Temperatur, umfassend zwischen 2 °C und 6 °C, danach Inkubieren des erhaltenen Reaktionsgemischs bei einer Temperatur, umfassend zwischen 35 °C und 40 °C, für eine Zeit, umfassend zwischen 30 Minuten und 2 Stunden;

c) Abkühlen des am Ende von Schritt b) erhaltenen Reaktionsgemischs auf eine Temperatur, umfassend zwischen 0 °C und 4 °C, und in der Gegenwart von EDTA;

d) Messen der Menge von Fragment C5a in dem abgekühlten in Schritt c) erhaltenen Reaktionsgemisch.

2. Verfahren nach Anspruch 1, **dadurch gekennzeichnet, dass** das Verhältnis der Menge des IgG in A in Gramm : Volumen nativem Serum in B in Liter aus Schritt b) zwischen 35 und 70 umfasst.

3. Verfahren nach Anspruch 2, **dadurch gekennzeichnet, dass** das Verhältnis der Menge des IgG in A in Gramm : Volumen nativem Serum in B in Liter aus Schritt b) zwischen 40 und 65 umfasst.

4. Verfahren nach Anspruch 2 oder 3, **dadurch gekennzeichnet, dass** das Verhältnis der Menge des IgG in A in Gramm : Volumen nativem Serum in B in Liter aus Schritt b) zwischen 45 und 60 umfasst.

5. Verfahren nach einem der Ansprüche 1 bis 4, **dadurch gekennzeichnet, dass** das Inkubieren von Schritt b) bei einer Temperatur von ungefähr 37°C während einer Zeit, die zwischen 45 Minuten bis 1 h umfasst, erfolgt.

6. Verfahren nach einem der Ansprüche 1 bis 5, **dadurch gekennzeichnet, dass** das EDTA in Schritt c) in einer Konzentration verwendet wird, umfassend zwischen 50 mM und 200 mM.

7. Verfahren nach Anspruch 6, **dadurch gekennzeichnet, dass** das EDTA in Schritt c) in einer Konzentration von etwa 100 mM verwendet wird.

8. Verfahren nach einem der Ansprüche 1 bis 7, **dadurch gekennzeichnet, dass** die Probe A eine IVIG-Probe ist.

9. Verfahren nach einem der Ansprüche 1 bis 8, **dadurch gekennzeichnet, dass** das native Serum der Probe B ein humanes Serum der Blutgruppe AB+ ist.

10. Verfahren nach einem der Ansprüche 1 bis 9, **dadurch gekennzeichnet, dass** es einen Schritt e) des Vergleichens der in d) erhaltenen Menge mit der Menge umfasst, die erhalten wird bei Substitution der Probe A durch Lipopolysaccharide (LPS) von E.Coli des Serotyps 0127 :B8, gereinigt durch Extraktion mit Phenol.

11. Verfahren nach einem der Ansprüche 1 bis 9, **dadurch gekennzeichnet, dass** es einen Schritt e) des Messens des Prozentsatzes der Komplementaktivierung umfasst, erhalten durch die nachfolgende Formel:

[Test - Negativkontrolle]/[maximale Komplementaktivierung - Negativkontrolle] * 100,
worin: "Test" die Menge des Fragments C5a ist, die in dem getesteten Reaktionsgemisch gemessen wird,
"Negativkontrolle" die Menge des Fragments C5a ist, die in dem Reaktionsgemisch gemessen wird, in welchem die Probe A durch Verdünnungspuffer ersetzt ist;
"Maximale Komplementaktivierung" die Menge des Fragments C5a ist, die in dem Reaktionsgemisch gemessen wird, in welchem die Probe A durch Lipopolysaccharide (LPS) von E.Coli des Serotyps 0127 :B8, gereinigt durch Extraktion mit Phenol, ersetzt ist.

12. Verfahren nach Anspruch 10 oder 11, **dadurch gekennzeichnet, dass** das Verhältnis der Menge des LPS in Gramm : Volumen nativem Serum in B in Liter zwischen 6 und 15 umfasst.

13. Verfahren nach Anspruch 12, **dadurch gekennzeichnet, dass** das Verhältnis der Menge des LPS in Gramm : Volumen nativem Serum in B in Liter zwischen 7 und 14 umfasst.

14. Verfahren nach Anspruch 12 oder 13, **dadurch gekennzeichnet, dass** das Verhältnis der Menge des LPS in Gramm : Volumen nativem Serum in B in Liter zwischen 8 und 13 umfasst.

15. Verfahren nach einem der Ansprüche 1 bis 14, **dadurch gekennzeichnet, dass** Schritt d) der Messung der Menge des Fragments C5a durch einen ELISA-Test durchgeführt wird.

**Claims**

1. A method for measuring immunoglobulin G-mediated complement activation, comprising the following steps:

   a) preparing a sample A of immunoglobulin G (IgG) and a sample B comprising native serum, said native serum optionally being diluted in a dilution buffer;
   b) mixing sample A with sample B at a ratio of "amount of IgG in A in grams" : "volume of native serum in B in liters" of between 30 and 75, at a temperature of between 2°C and 6°C, and subsequently incubating the resulting reaction mixture at a temperature of between 35°C and 40°C for a period of between 30 minutes and 2 hours;
   c) cooling the reaction mixture obtained at the end of step b) to a temperature of between 0°C and 4°C in the presence of EDTA;
   d) measuring the amount of C5a fragment in the cooled reaction mixture obtained in c).

2. Method according to Claim 1, **characterized in that** the ratio of "amount of IgG in A in grams" : "volume of native serum in B in liters" of step b) is between 35 and 70.

3. Method according to Claim 2, **characterized in that** the ratio of "amount of IgG in A in grams" : "volume of native serum in B in liters" of step b) is between 40 and 65.

4. Method according to Claim 2 or 3, **characterized in that** the ratio of "amount of IgG in A in grams" : "volume of native serum in B in liters" of step b) is between 45 and 60.

5. Method according to one of Claims 1 to 4, **characterized in that** the incubation of step b) is carried out at a temperature of approximately 37°C, for a period of between 45 minutes and 1h.

6. Method according to any one of Claims 1 to 5, **characterized in that** the EDTA is used in step c) at a concentration of between 50 mM and 200 mM.

7. Method according to Claim 6, **characterized in that** the EDTA is used in step c) at a concentration of approximately 100 mM.

8. Method according to any one of Claims 1 to 7, **characterized in that** sample A is a sample of IVIG.

9. Method according to any one of Claims 1 to 8, **characterized in that** the native serum of sample B is a human serum of blood group AB+.

10. Method according to any one of Claims 1 to 9, **characterized in that** it comprises a step e) of comparing the amount obtained in d) with the amount obtained by replacing sample A with lipopolysaccharides (LPS) of E.coli serotype 0127 :B8, purified by extraction with phenol.

11. Method according to any one of Claims 1 to 9, **characterized in that** it comprises a step e) of measuring the percentage of complement activation, obtained by means of the following formula:

```
[test - negative control] / [maximum complement
activation - negative control] × 100
```

in which:

   "test" is the amount of C5a fragment measured in the reaction mixture tested;
   "negative control" is the amount of C5a fragment measured in the reaction mixture in which sample A is replaced with the dilution buffer;
   "maximum complement activation" is the amount of C5a fragment measured in the reaction mixture in which sample A is replaced with lipopolysaccharides (LPS) of E.coli serotype 0127 :B8, purified by extraction with phenol.

12. Method according to Claim 10 or 11, **characterized in that** the ratio of "amount of LPS in grams" : "volume of native serum in B in liters" is between 6 and 15.

13. Method according to Claim 12, **characterized in that** the ratio of "amount of LPS in grams" : "volume of native serum in B in liters" is between 7 and 14.

14. Method according to Claim 12 or 13, **characterized in that** the ratio of "amount of LPS in grams" : "volume of native serum in B in liters" is between 8 and 13.

15. Method according to any one of Claims 1 to 14, **characterized in that** step d) of measuring the amount of C5a fragment is carried out by means of an ELISA assay.

# Figure 1

DO des différents LPS

## Figure 2

## RÉFÉRENCES CITÉES DANS LA DESCRIPTION

**Documents brevets cités dans la description**

- WO 2007077365 A

**Littérature non-brevet citée dans la description**

- **SPYCHER M et al.** In vitro comparison of the complement-scavenging capacity of different intravenous immunoglobulin preparations. *VOX SANGUINIS,* 2009, vol. 97 (4), 348-354